# DEMANDE DE BREVET EUROPEEN

(11) **EP 1 493 444 A1**
(43) Date de publication de la demande: **05.01.2005**
(21) Numéro de dépôt: 04360043.6
(22) Date de dépôt: 23.04.2004
(51) Int. Cl.: A61K 31/785

(54) **Composition pharmaceutique utilisée en tant qu'agent anti-infectieux des voies respiratoires supérieures**

(30) Priorité: 20.06.2003 FR 0307460
(71) Demandeur: Anben Pharma SARL, 67300 Schiltigheim (FR)
(72) Inventeur: Andermann, Guy, 67000 Strasbourg (FR)

(57) **Abrégé**

Composition pharmaceutique utilisée en tant qu'agent anti-infectieux des voies respiratoires supérieures.
Elle comprend :
- un pourcentage (A) compris entre 0,0005 % et 0,5 % en masse de polihexanide,
- un pourcentage (B) compris entre 0,2 % et 10 % en masse de dexpanthénol,
- un pourcentage (C) compris entre 0,05 % et 2% en masse de polysorbate 80,
- un pourcentage (D) compris entre 25 % et 50 % en masse d'eau de mer,
- un pourcentage (E) d'eau purifiée, ajusté de sorte à ce que (A) +(B) +(C) +(D) + (E) représente 100% en masse de la composition.

## Description

La présente invention concerne une composition pharmaceutique destinée à être utilisée au niveau des voies respiratoires supérieures en tant qu'agent anti-infectieux, pour traiter des affections, telles que notamment des rhumes.

A l'heure actuelle, on utilise à cet effet essentiellement deux types de médicaments, dont le premier repose sur des compositions comprenant des molécules à propriétés antibiotiques, tandis que le second repose sur des compositions comprenant des molécules de la famille des ammoniums quaternaires.

Bien que les antibiotiques soient particulièrement efficaces dans la plupart des cas, L'Organisation Mondiale pour la Santé recommande depuis plusieurs années de modérer leur administration, afin de limiter les réactions de résistance croisées que leur utilisation massive suscite auprès des microbes, et qui constituent d'ores et déjà, au niveau mondial, un réel problème de santé publique, bien connu des spécialistes.

Par ailleurs, l'on a pu constater que les molécules de la famille des ammoniums quaternaires agressent la peau et les muqueuses, en particulier lorsqu'elles sont administrées sur une période continue, et peuvent être à l'origine de lésions de la muqueuse nasale parfois très gênantes dans des situations de rhume allergique ou infectieux.

La présente invention a par conséquent pour but de pallier ces inconvénients en proposant une composition pharmaceutique qui soit à la fois efficace pour combattre les micro-organismes pathogènes, peu agressive aussi bien vis à vis de l'utilisateur que de l'environnement, et d'utilisation aisée.

Une telle composition se caractérise en ce qu'elle comprend :
- un pourcentage (A) compris entre 0,0005 % et 0,5 % en masse de polihexanide,
- un pourcentage (B) compris entre 0,2 % et 10g% en masse de dexpanthénol,
- un pourcentage (C) compris entre 0,05 % et 2% de polysorbate 80,
- un pourcentage (D) compris entre 25 % et 50 % en masse d'eau de mer, et
- un pourcentage (E) d'eau purifiée, ajusté de sorte à ce que (A) +(B) +(C) +(D) + (E) représente 100% en masse de la composition.

Selon une formulation préférentielle, la composition selon l'invention se caractérise encore en ce qu'elle comprend :
- un pourcentage (A) compris entre 0,005 % et 0,0075 % en masse de polihexanide,
- un pourcentage (B) compris entre 1,5 % et 2,5 % en masse de dexpanthénol,
- un pourcentage (C) compris entre 0,10 % et 0,3 % en masse de polysorbate 80,
- un pourcentage (D) compris entre 31 % et 35 % d'eau de mer,
- un pourcentage (E) d'eau purifiée ajusté de sorte à ce que (A) +(B) +(C)+ (D) +(E) représente 100 % en masse de la composition.

Une telle formulation a ainsi pour principal avantage de combiner à la fois les propriétés antiseptiques du polihexanide, et cicatrisantes du dexpanthénol, lesquels peuvent en outre agir de manière optimale, grâce à la présence du polysorbate qui, en libérant la muqueuse nasale des impuretés, contribue à un parfait contact entre les zones à traiter et les principes actifs.

La présente composition pharmaceutique peut encore se
caractériser en ce qu'elle comprend un pourcentage (F) d'additifs chimiques destinés à améliorer son goût et/ou son odeur, le pourcentage (E) d'eau purifiée étant alors ajusté de sorte à ce que (A) + (B) + (C) +(D) + (E) + (F) représente 100 % en masse de la composition.

Elle peut à cet effet contenir des huiles essentielles, par exemple des huiles essentielles de menthe ou de rose, ou des extraits d' huiles essentielles, par exemple du menthol, particulièrement agréables en cas de nez congestionné.

D'autre part, la présente invention concerne également l'application d'une telle composition pharmaceutique, au traitement des infections des voies respiratoires supérieures, ainsi que son utilisation par pulvérisation au niveau des voies respiratoires supérieures au moyen d'un diffuseur de type spray nasal.

Il peut d'ailleurs être noté à ce sujet, que ladite composition est parfaitement adaptée pour être conditionnée dans un diffuseur de type valve poche fonctionnant non seulement sans gaz fluorés nocifs pour l'environnement, mais permettant en outre avantageusement une diffusion homogène des principes actifs quelle que soit sa position, et pouvant être adaptée à une utilisation chez les nourrissons et les enfants en bas âge par un réglage de la pression.

Quelques résultats expérimentaux permettront à présent de mieux comprendre les avantages et les propriétés de la composition pharmaceutique selon la présente invention.

Un des essais réalisés a consisté à évaluer le potentiel cytotoxique de la composition, déterminé 48 heures après son application sur des cellules ensemencées en microplaque de 96 puits.

Les paramètres d'appréciation de la cytotoxicité retenus ont été le pourcentage de mortalité cellulaire et la concentration du produit inhibant de 50 % (CI50), ainsi que la survie et la croissance cellulaire.

Une CI50 de 10,3 % a ainsi pu être déterminée, ce qui correspond à une cytotoxicité avantageusement modérée.

Un autre essai a consisté à évaluer le risque de sensibilisation de la composition, appliquée par voie cutanée auprès de 10 cobayes.

L'effet recherché a, par ailleurs, été accentué par injection d'Adjuvant Complet de Freund.

Les 10 cobayes traités ont été exposés à la composition selon l'invention par injection intradermique à la concentration de 100 % dans l'eau PPI, ainsi que par application topique à la concentration de 100 %.

En parallèle, 5 cobayes témoins se sont vus administrer uniquement le solvant en injection intradermique et en application topique.

Après une période de repos de 10 à 14 jours qui achève la période d'induction, tous les animaux ont été exposés à la dose de déclenchement non irritante de 100 %.

L'étendue et le degré de la réaction cutanée des animaux traités ont été comparés à ceux de la réaction observée chez les animaux témoins.

La composition selon l'invention a ensuite été classée en fonction du pourcentage d'animaux réactifs, conformément à la norme NF EN ISO 10993-10-mars 1996 concernant l'évaluation biologique des dispositifs médicaux.

Les résultats ont montré qu'aucun des cobayes traités ou des cobayes témoins n'était réactif.

La composition selon l'invention a donc pu être classée faiblement (voire non significativement) sensibilisante par contact avec la peau.

Un autre essai a encore consisté à apprécier la tolérance nasale de la composition selon l'invention après applications itératives pendant 14 jours consécutifs chez le lapin.

En fait, l'objectif de cet essai a été d'évaluer la tolérance locale au niveau de la muqueuse nasale et de l'éventuelle toxicité générale de la composition.

Pour cela, plusieurs animaux ont reçu, par jour, dans chaque narine, 0,1 ml de la composition telle quelle, alors que d'autres animaux, servant de témoins, ont été traités dans les mêmes conditions avec du NaCl à 0,9%.

Les animaux ont été observés régulièrement au cours de l'essai, et tous les effets locaux ou généraux ont été enregistrés.

Au terme de l'essai, un examen macroscopique de la muqueuse nasale (avec observation de l'activité ciliaire), ainsi qu'un examen histologique des fosses nasales, de la trachée et des poumons ont été effectués chez tous les animaux.

Les résultats ont été exprimés sous forme d'un score d'irritation moyen (ScM), et les valeurs obtenues ont été comparées à celles figurant ci-après dans le tableau 1.

Le degré d'irritation locale de la muqueuse a été apprécié en fonction du score d'irritation moyen obtenu et la tolérance locale de la composition a été appréciée comparativement à celle du NaCl à 0,9 %.

**Tableau 1 :**

| Score d'irritation moyen (ScM) | Degré d'irritation de la muqueuse |
|---|---|
| 0<ScM<6 | Absence d'irritation significative |
| 6≤ScM<11 | Irritation légère |
| 11≤ScM<16 | Irritation modérée |
| 16≤ScM≤22 | Irritation grave |

Pour les animaux témoins on a déterminé un ScM de 3,1, tandis que pour les animaux sur lesquels la composition selon l'invention a été testée, on a une déterminé un ScM de 2,2, ce qui correspond, au vu du tableau 1, a une absence d'irritation significative.

Par conséquent, la composition selon l'invention a pu être appliquée chez le lapin 2 fois par jours pendant 14 jours consécutifs sans entraîner aucune irritation significative au niveau de la muqueuse nasale.

Enfin, une autre expérience a encore consisté à comparer la toxicité aiguë in vivo du polihexanide avec celle d'autres composés.

A cet effet, la toxicité du polihexanide a été comparée in vivo chez le lapin à celle du digluconate de chlorhexidine, et à celle de trois chlorures de benzyldiméthylalkylammonium.

Pour chacune des substances étudiées, une concentration théorique provoquant une irritation de la muqueuse moyenne identique (C₂₀) a été déterminée.

Ces concentrations sont respectivement :
- 0,25 g % pour le chlorure de benzalkonium
- 0,28 g % pour le chlorure de benzyldiméthyldodecylammonium
- 0,32 g % pour le chlorure de benzyldiméthylhexadecylanmonium
- 0,54 g % pour le digluconate de chlorhexidine
- 12,2 g % pour le polihexanide.

Ces valeurs montrent par conséquent, que le polihexanide est beaucoup moins toxique pour la muqueuse que les autres composés testés, puisque sa valeur C₂₀ est 22 fois supérieure à celle de la chlorhexidine et 44 fois plus élevée que la valeur moyenne des chlorures de benzalkonium.

Enfin, l'intérêt du polihexanide prend toute sa signification si l'on considère qu'il est utilisé à des concentrations 25 à 50 fois plus faible que le digluconate de chlorhexidine et le chlorure de benzalkonium.

Tel qu'il ressort clairement de ces différentes expérimentations, la composition selon l'invention présente de nombreux avantages par rapport aux produits couramment utilisés pour la même application.

En particulier, elle présente une faible cytotoxicité, et n'est ni agressive ni toxique vis à vis de la peau et des muqueuses.

## Revendications

1. Composition pharmaceutique utilisée en tant qu'agent anti-infectieux des voies respiratoires supérieures, **caractérisée en ce qu'**elle comprend :
- un pourcentage (A) compris entre 0,0005 % et 0,5 % en masse de polihexanide,
- un pourcentage (B) compris entre 0,2 % et 10 % en masse de dexpanthénol,
- un pourcentage (C) compris entre 0,05 % et 2% en masse de polysorbate 80,
- un pourcentage (D) compris entre 25 % et 50 % en masse d'eau de mer,
- un pourcentage (E) d'eau purifiée, ajusté de sorte à ce que (A) +(B) +(C) +(D) + (E) représente 100% en masse de la composition.

2. Composition pharmaceutique selon la revendication 1,
**caractérisée en ce qu'**elle comprend :
- un pourcentage (A) compris entre 0,005 % et 0,0075 % en masse de polihexanide,
- un pourcentage (B) compris entre 1,5 % et 2,5 % en masse de dexpanthénol,
- un pourcentage (C) compris entre 0,10 % et 0,3 % en masse de polysorbate 80,
- un pourcentage (D) compris entre 31 % et 35 % d'eau de mer,
- un pourcentage (E) d'eau purifiée ajusté de sorte à ce que (A) +(B) +(C)+ (D) +(E) représente 100 % en masse de la composition.

3. Composition pharmaceutique selon l'une quelconque des revendications précédentes, **caractérisée en ce qu'**elle comprend un pourcentage (F) d'additifs chimiques destinés à améliorer son goût et/ou son odeur, le pourcentage (E) d'eau purifiée étant alors ajusté de sorte à ce que (A) + (B) + (C) +(D) + (E) + (F) représente 100 % en masse de la composition.

4. Composition pharmaceutique selon la revendication 3, **caractérisée en ce qu'**elle comprend des huiles essentielles, par exemple des huiles essentielles de menthe ou de rose, ou des extraits d' huiles essentielles, par exemple du menthol.

5. Application d'une composition pharmaceutique selon l'une quelconque des revendications précédentes, au traitement des infections des voies respiratoires supérieures.

6. Utilisation d'une composition pharmaceutique selon l'une quelconque des revendications précédentes, par pulvérisation au niveau des voies respiratoires supérieures.
